# EUROPEAN PATENT SPECIFICATION

(11) **EP 1 097 917 B1**
(45) Date of publication and mention of the grant of the patent: **12.02.2003**
(21) Application number: 99120951.1
(22) Date of filing: 03.11.1999
(51) Int. Cl.: C07C 67/313, C07C 69/716, C07C 69/738, C07C 51/373, C07C 59/84

(54) **Preparing method of alpha-ketocarboxylic acid derivatives**
Verfahren zum Herstellen von Derivaten von Alpha-Ketocarbonsäuren
Procédé de préparation de dérivés d'acide alpha-cétocarboxylique

(43) Date of publication of application: 09.05.2001
(73) Proprietor: KOREA RESEARCH INSTITUTE OF CHEMICAL TECHNOLOGY, Daejeon, 305-543 (KR)
(72) Inventor: Kim, Dae Whang, Daejeon, 305-333 (KR); Chang, Hae Sung, Daejeon, 305-333 (KR); Ko, Young Kwan, Daejeon, 305-333 (KR); Ryu, Jae Wook, Daejeon, 305-333 (KR); Woo, Jae Chun, Daejeon, 302-162 (KR); Koo, Dong Wan, Daejeon, 305-333 (KR)
(74) Representative: Reinhard - Skuhra - Weise & Partner

(56) References cited:
- EP-A- 0 011 207
- HIROFUMI MAEKAWA ET AL.: "Facile synthesis of alpha-Keto Carbonyl Compounds by Indirect Anodic Oxydation" CHEMISTRY LETTERS., no. 6, 1994, pages 1017-1020, XP002133808 CHEMICAL SOCIETY OF JAPAN. TOKYO., JP ISSN: 0366-7022

## Description

### BACKGROUND OF THE INVENTION

### Field of the Invention

The present invention relates to a method for preparing α-ketocarboxylic acid derivatives, more specifically to a method for preparing α-ketocarboxylic acid derivatives, expressed by the following formula 1, which are widely used as major source material for the synthesis of agrochemicals and pharmaceutical drugs, by reacting α-hydroxycarboxylic acid derivatives with hypobromous acid (HOBr), 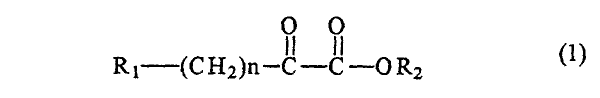 wherein R₁ represents a hydrogen atom, C₁ ∼ C₁₀ alkyl, C₁ ∼ C₁₀ haloalkyl, C₁ ∼ C₁₀ alkoxyalkyl, C₃ ∼ C₆ cycloalkyl or aryl, wherein the aryl is benzene, naphthalene, pyridine or thiophene, the aryl having more than one of the group consisting of hydrogen atom and the functional groups hydroxy, halogen atom, C₁ ∼ C₃ alkyl, -(CH₂)ₘL, C₁ ∼ C₃ alkoxy, phenoxy and nitro group, wherein the L is a halogen atom, alkylsulphonyloxy, benzenesulfonyloxy or toluenesulfonyloxy group, and the m is an integer of 1 or 2; R₂ represents a hydrogen atom or C₁ ∼ C₆ alkyl; and n represents 0 or an integer of 1 ∼ 10.

α-Ketocarboxylic acid derivatives expressed by formula 1 are widely known and used as major source material for the synthesis of various agrochemicals and pharmaceutical drugs [German patent 2,624,174; *Syn. Commun*., **26,** 2229 (1996), *Huaxi Yaoue Zazhi*, **11,** 221(1996)].

Also, the preparing methods of α-ketocarboxylic acid derivatives expressed by formula 1 are being developed extensively, and some representative examples are disclosed in EP 412,378, *Org. Syn. Coll.* vol. 4, 467(1963), *Tetraedron Lett.,* 33(47), 7245(1992) and *Syn. Commun*., 26(11), 2229(1996).

In the conventional preparing methods, the reactions are performed using NaOCl, KMnO₄, oxaziridine or CrO₃ as oxidizing agents as in the following schemes 1 ∼ 4.

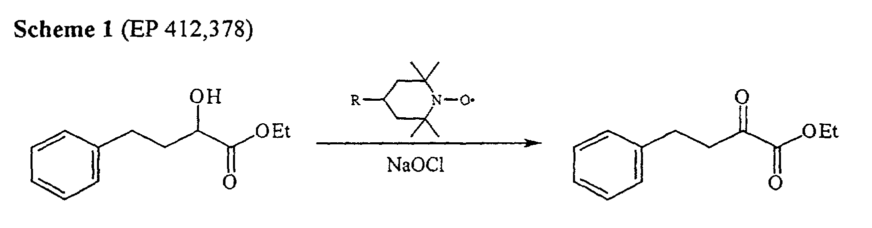

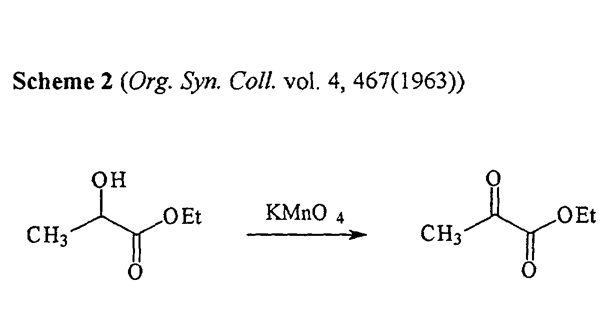

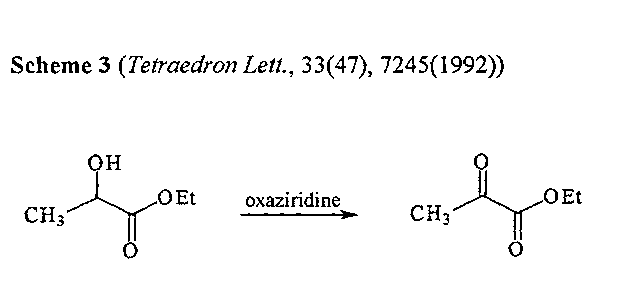

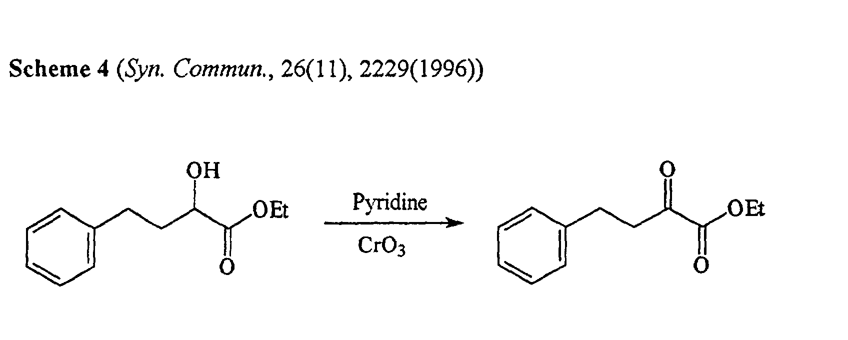

However, the above conventional preparing methods are undesirable in that oxidants harmful to humans and the environment, such as potassium permanganate (KMnO₄) and chromium trioxide (CrO₃), or expensive reagents as nitroxy radicals and oxaziridine are used.

Accordingly, an object of this invention is to provide an industrially advantageous preparing method of α-ketocarboxylic acid derivatives from α-hydroxycarboxylic acid derivatives in the presence of hypobromous acid.

### SUMMARY OF THE INVENTION

The inventors made extensive efforts to solve the problems of the conventional preparing methods. As a result, it was realized that the use of hypobromous acid instead of environmentally harmful or expensive oxidants could solve all the said problems.

### Detailed Description of the Invention

The present invention is characterized by a preparing method of α-ketocarboxylic acid derivatives expressed by the following formula 1, wherein the starting material (α-hydroxycarboxylic acid derivatives) expressed by the following formula 2 is reacted with hypobromous acid as an oxidant, 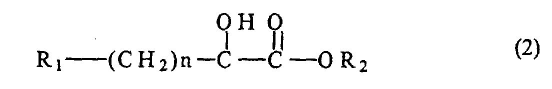 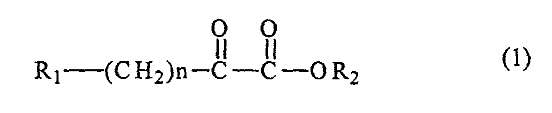 wherein R₁, R₂ and n are the same as explained above.

The detailed description of the present invention is given hereunder.

The present invention excludes the use of potassium permanganate and chromium trioxide, disclosed in *Org. Syn. Coll*. vol. 4, 467(1963) and *Syn. Commun*., 229(1996), which are harmful to humans and the environment. Also, the present invention excludes the use of expensive compounds such as oxaziridine and nitroxy radicals as disclosed in *Tetrahedron Lett.* 7245(1992) and EP 412,378. Therefore, the present invention provides a safe preparing method. Also, the oxidation of α-hydroxycarboxylic acid derivatives is performed in a mild reaction condition and little by-product is produced, so that the target product is obtained in good yield.

The preparation of α-ketocarboxylic acid derivatives expressed by formula 1 according to the present invention can be simplified by the following scheme 5, 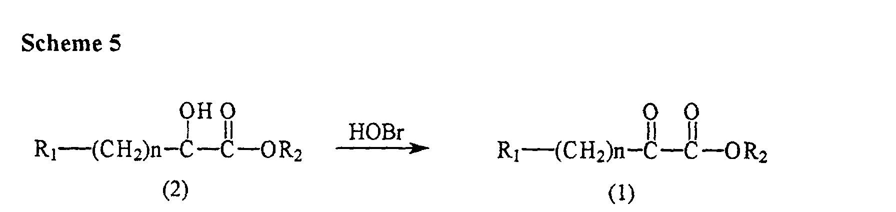 wherein R₁, R₂ and n are the same as explained above.

α-Hydroxycarboxylic acid derivatives expressed by formula 2, used as a starting material of the present invention, are well known compounds and can be purchased inexpensively or prepared easily using the known preparing methods.

The reaction of α-hydroxycarboxylic acid derivatives expressed by formula 2 with hypobromous acid to obtain α-ketocarboxylic acid derivatives expressed by formula 1 is performed in the range of -10 ∼ 50°C, preferably in the range of -5 ∼ 40°C. Hypobromous acid is used in the range of 1.0 ∼ 2.0 molar equivalents to α-hydroxycarboxylic acid derivatives expressed by formula 2, and preferably in the range of 1.05 ∼ 1.3 molar equivalents to α-hydroxycarboxylic acid derivatives expressed by formula 2. It is preferred to use halogenated hydrocarbon as a solvent such as dichloromethane, chloroform, carbon tetrachloride and 1,2-dichloroethane.

After oxidation, unreacted hypobromous acid can be decomposed easily by treatment with salts of thiosulfate. If necessary, high purity of the desired product can be obtained by conventional methods.

Hypobromous acid is widely known as an oxidizing biocide for water treatment and can be generated easily from sodium hypobromite, potassium hypobromite, sodium hypochlorite and potassium hypochlorite. Some exemplary compounds used for generating hypobromous acid from sodium hypobromite and potassium hypobromite are bicarbonates such as sodium bicarbonate and potassium bicarbonate, inorganic acids such as hydrochloric acid, sulfuric acid, nitric acid, boric acid and phosphoric acid, and organic acids such as acetic acid and propionic acid. In addition, hypobromous acid can be easily obtained by reacting salts of hypochlorous acid with HBr [USP 5,641,520].

Sodium hypobromite and potassium hypobromite for generating hypobromous acid can be generated easily by reacting 2 equivalents of sodium hydroxide or potassium hydroxide with 1 equivalent of bromine in aqueous solution in the range of -5 ∼ 0°C [*Org. Syn. Coll*., 5, 8 (1973)].

The following examples are intended to be illustrative of the present invention.

### Example 1: Preparation of Pyruvic Acid Butyl Ester (Compound No.1)

After putting 700ml of water and 96g (2.4mol) of sodium hydroxide into a 2ℓ three-necked flask equipped with cooler, mechanical stirrer and thermometer, the reaction mixture was cooled to -5°C. 62ml (1.2mol) of bromine was added into a dropping funnel and dropped slowly to the sodium hydroxide solution for 2 hours, keeping the internal temperature at -5°C to generate sodium hypobromite (NaOBr). 700ml of dichloromethane and 148.12ml (1mol) of α-hydroxypropionic acid butyl ester were added slowly to the aqueous sodium hypobromite solution so that the temperature did not exceed 0°C. 26.1ml (0.3mol) of concentrated hydrochloric acid was added dropwise for 15 minutes, keeping the temperature of the reaction mixture at 0°C. After removing the cooler, the reaction mixture was stirred for 5 hours at room temperature. After adding sodium thiosulfate (100ml of water and 124g of Na₂S₂O₃·5H₂O) to the reaction mixture, the reaction mixture was stirred for 15 minutes. The organic layer was separated, and the aqueous layer was extracted twice with dichloromethane. The combined organic layers were dried over magnesium sulfate and filtered. The filtrate was evaporated and distilled off by fractional distillation under reduced pressure 0.027 bar (20 mm Hg), b.p. = 83 ∼ 86°C) to give 135.5g of oily target compound (94% yield).
¹H NMR (CDCl₃): δ 4.25 (t, 2H), 2.46 (s, 3H), 1.72 (m, 2H), 1.43 (m, 2H), 0.95 (t, 3H)

### Example 2: Preparation of Pyruvic Acid Butyl Ester

After putting 700ml of water and 96g (2.4mol) of sodium hydroxide into a 2ℓ three-necked flask equipped with cooler, mechanical stirrer and dropping funnel, the reaction mixture was cooled to -5°C. 62ml (1.2mol) of bromine was added into the dropping funnel and dropped slowly to the sodium hydroxide solution for 2 hours, keeping the internal temperature at -5°C to generate sodium hypobromite (NaOBr).

700ml of dichloromethane and 148.12ml (1mol) of α-hydroxypropionic acid butyl ester were added slowly to the aqueous sodium hypobromite solution so that the temperature did not exceed 0°C. 16.8g (0.2mol) of sodium bicarbonate dissolved in 100 ml of water was added dropwise slowly, keeping the temperature of the reaction mixture at 0°C. After removing the cooler, the reaction mixture was stirred for 5 hours at room temperature. After adding sodium thiosulfate (100ml of water and 124g of Na₂S₂O₃·5H₂O) to the reaction mixture, the reaction mixture was stirred for 15 minutes. The organic layer was separated, and the aqueous layer was extracted twice with dichloromethane. The combined organic layers were dried over magnesium sulfate and filtered. The filtrate was evaporated and distilled off by fractional distillation under reduced pressure (0.027 bar (20 mm Hg), b.p. = 83 ∼ 86°C) to give 132.5g (92% yield) of oily target compound.

### Example 3 : Preparation of Benzoyl Formic Acid(Compound No. 2)

After putting sodium hypobromite solution (17.5ml of water and 3.54g (0.03mol) of NaOBr) into a 50ml three-necked flask, the temperature was lowered to -5°C. 17.5ml of dichloromethane and 3.8g (0.025mol) of mandelic acid were added slowly, keeping the temperature of the reaction mixture in the range of -5∼0°C.

0.65ml of concentrated hydrochloric acid was added dropwise for 5 minutes, keeping the temperature of the reaction mixture at 0°C. After removing the cooler, the reaction mixture was stirred for 5 hours at room temperature. After adding sodium thiosulfate (5ml of water and 1.5g of Na₂S₂O₃·5H₂O) to the reaction mixture, the reaction mixture was stirred for 15 minutes. The organic layer was separated, and the aqueous layer was extracted twice with dichloromethane. The combined organic layers were dried over magnesium sulfate, and 3.41g (91% yield) of the target compound (m.p. = 64 ∼ 66°C) was obtained by evaporation.

### Example 4 : Preparation of Ethyl-2-Oxo-4-Phenyl Butyrate(Compound No. 3)

After inserting aqueous sodium hypobromite solution (17.5ml of water and 3.54g (0.03mol) of NaOBr) in a 50ml three-necked flask, the temperature was lowered to -5°C. 17.5ml of dichloromethane and 5.2g (0.025mol) of 2-hydroxy-4-phenylbutyric acid ethyl ester were added slowly, keeping the temperature of the reaction mixture in the range of -5∼0°C. 0.65ml of concentrated hydrochloric acid was added dropwise for 5 minutes, keeping the temperature of the reaction mixture at 0°C. After removing the cooler, the reaction mixture was stirred for 5 hours at room temperature. After adding sodium thiosulfate (5ml of water and 1.5g of Na₂S₂O₃·5H₂O) to the reaction mixture, the reaction mixture was stirred for 15 minutes. The organic layer was separated, and the aqueous layer was extracted twice with dichloromethane. The combined organic layers were dried over magnesium sulfate, evaporated and purified by column chromatography to obtain 4.74g (92% yield) of the target compound.
¹HNMR (CDCl₃): 7.2 (m, 5H), 4.3 (q, 2H), 3.1 (t, 2H), 2.9 (t, 2H), 1.3 (t, 3H)

- With the same method as described in Example 1 ∼ 4, for example the following further α-ketocarboxylic acid derivatives were prepared from α-hydroxycarboxylic acid derivatives. The results are shown in the following Table 1.

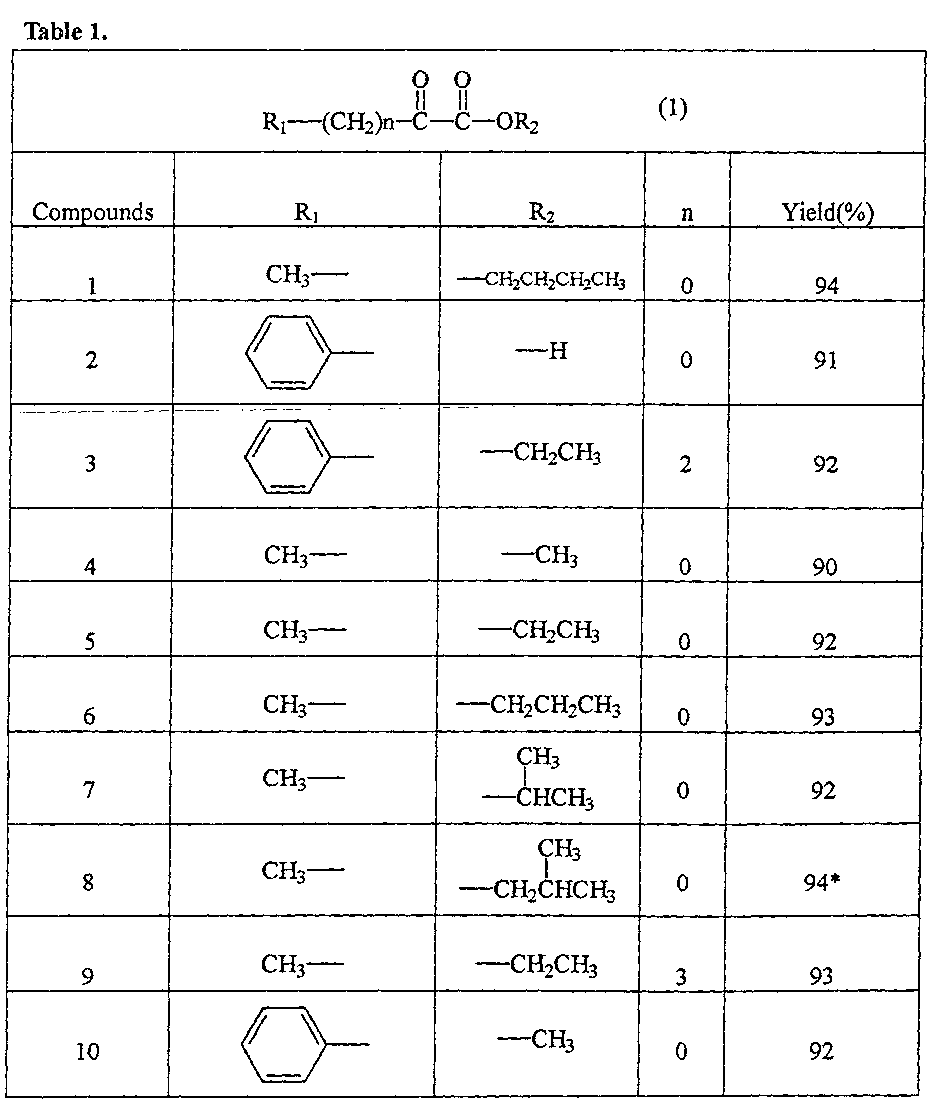

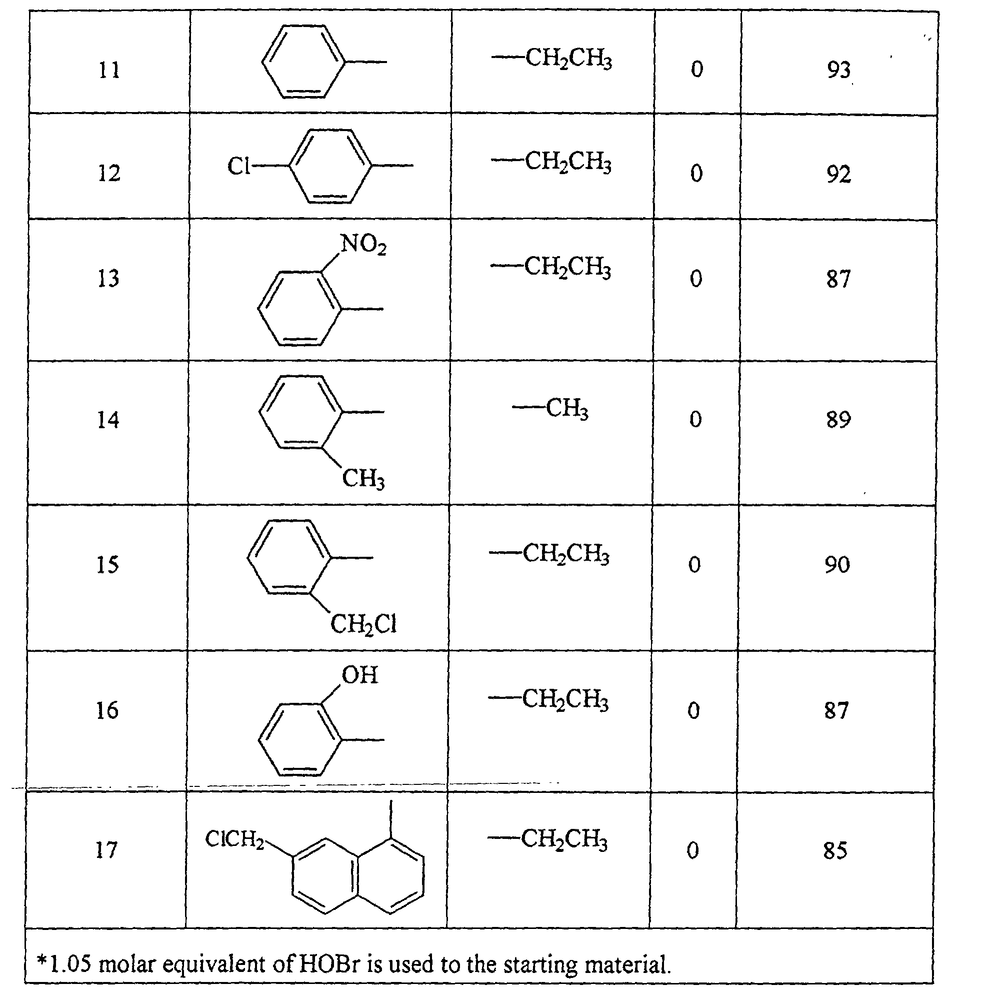

As described above, the present invention provides an industrially advantageous preparing method of α-ketocarboxylic acid derivatives from α-hydroxycarboxylic acid derivatives in the presence of hypobromous acid.

## Claims

1. A method for preparing α-ketocarboxylic acid derivatives expressed by the following formula 1 by reacting α-hydroxycarboxylic acid derivatives expressed by the following formula 2 with an oxidizing agent,
**characterized in that** the oxidizing agent is hypobromous acid, 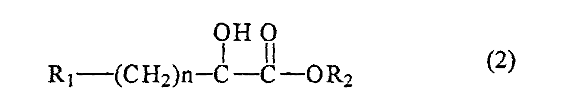 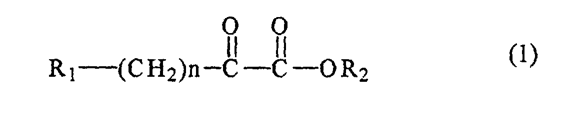 wherein R₁ represents a hydrogen atom, C₁ ∼ C₁₀ alkyl, C₁ ∼ C₁₀ haloalkyl, C₁ ∼ C₁₀ alkoxyalkyl, C₃ ∼ C₆ cycloalkyl or aryl, wherein the said aryl is benzene, naphthalene, pyridine or thiophene, the aryl having more than one of the group consisting of hydrogen atom and the functional groups hydroxy, halogen atom, C₁ ∼ C₃ alkyl, -(CH₂)ₘL, C₁ ∼ C₃ alkoxy, phenoxy and nitro group, wherein the said L is a halogen atom, alkylsulphonyloxy, benzenesulfonyloxy or toluenesulfonyloxy group, and the said m is an integer of 1 or 2; R₂ represents a hydrogen atom or C₁ ∼ C₆ alkyl; and n represents 0 or an integer of 1 ∼ 10.

2. The method according to claim 1, wherein the said hypobromous acid is used in the range of 1 ∼ 2 molar equivalents to the starting material expressed by formula 2.

3. The method according to claim 1 or 2, wherein the said hypobromous acid is used in the range of 1.05 ∼ 1.3 molar equivalents to the starting material expressed by formula 2.

4. The method according to one or more of the preceding claims, wherein the reaction is performed in the temperature range of -5∼40°C.

5. The method according to one or more of the preceding claims, wherein the compound expressed by Formula 1 is pyruvic acid ethyl ester.

6. The method according to one or more of claims 1-4, wherein the compound expressed by Formula 1 is pyruvic acid butyl ester.

7. The method according to one or more of claims 1-4, wherein the compound expressed by Formula 1 is (2-methylphenyl)-glyoxylic acid methyl ester.

8. The method according to one or more of claims 1-4, wherein the compound expressed by Formula 1 is (2-chloromethylphenyl)-glyoxylic acid ethyl ester.

9. The method according to one or more of claims 1-4, wherein the compound expressed by Formula 1 is (2-hydroxyphenyl)-glyoxylic acid ethyl ester.

10. The method according to one or more of claims 1-4, wherein the compound expressed by Formula I is (7-chloromethylnaphthyl)-glyoxylic acid ethyl ester.

## Patentansprüche

1. Verfahren zur Herstellung von α-Ketocarbonsäure-Derivaten, ausgedrückt durch die nachfolgende Formel 1, durch Umsetzen von α-Hydroxycarbonsäurederivaten, ausgedrückt durch die nachfolgende Formel 2, mit einem Oxidationsmittel,
**dadurch gekennzeichnet, daß**
das Oxidationsmittel hypobromige Säure ist, 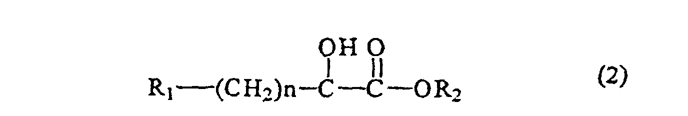 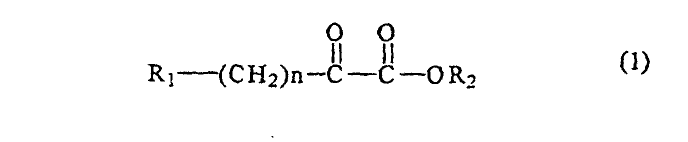 wobei R₁ ein Wasserstoffatom, C₁ ∼ C₁₀ Alkyl, C₁ ∼ C₁₀ Haloalkyl, C₁ ∼ C₁₀ Alkoxyalkyl, C₃ ∼ C₆ Cycloalkyl oder Aryl darstellt, wobei das Aryl Benzol, Naphthol, Pyridin oder Thiophen ist, wobei das Aryl mehr als Eines aus der Gruppe aufweist, die aus einem Wasserstoffatom und den funktionellen Gruppen Hydroxy-, Halogenatom, C₁ ∼ C₃ Alkyl, -(CH₂)ₘL, C₁ ∼ C₃ Alkoxy-,Phenoxy- und einer Nitro-Gruppe besteht, wobei L eine Halogenatom-, Alkylsulphonyloxy-, Benzolsulfonyloxy-, oder Toluolsulfonyloxy-Gruppe ist, und m eine ganze Zahl von 1 oder 2 ist; ein ein Wasserstoffatom oder C₁ ∼ C₆ Alkyl darstellt; und n 0 oder eine ganze Zahl von 1 ∼ 10 darstellt.

2. Verfahren nach Anspruch 1, wobei die hypobromige Säure in einem Bereich von 1 ∼ 2 molaren Äquivalenten zum durch Formel 2 ausgedrückten Ausgangsmaterial verwendet wird.

3. Verfahren nach Anspruch 1 oder 2, wobei die hypobromige Säure im Bereich von 1,05 ∼ 1,3 molaren Äquivalenten zum durch Formel 2 ausgedrückten Ausgangsmaterial verwendet wird.

4. Verfahren nach einem oder mehreren der vorhergehenden Ansprüche, wobei die Umsetzung in einem Temperaturbereich von -5 ∼ 40°C durchgeführt wird.

5. Verfahren nach einem oder mehreren der vorhergehenden Ansprüche, wobei die durch Formel 1 ausgedrückte Verbindung Brenztraubensäureethylester ist.

6. Verfahren nach einem oder mehreren der Ansprüche 1 - 4, wobei die durch Formel 1 ausgedrückte Verbindung Brenztraubensäurebutylester ist

7. Verfahren nach einem oder mehreren der Ansprüche 1 - 4, wobei die von Formel 1 ausgedrückte Verbindung (2-Methylphenyl)-glyoxalsäuremethylester ist.

8. Verfahren nach einem oder mehreren der Ansprüche 1 - 4, wobei die von Formel 1 ausgedrückte Verbindung (2-Chlormethylphenyl)-glyoxalsäureethylester ist.

9. Verfahren nach einem oder mehreren der Ansprüche 1 - 4, wobei die von Formel 1 ausgedrückte Verbindung (2-Hydroxyphenyl)-glyoxalsäureethylester ist.

10. Verfahren nach einem oder mehreren der Ansprüche 1 - 4, wobei die von Formel 1 ausgedrückte Verbindung (7-Chlormethylnaphthyl)-glyoxalsäureethylester ist.

## Revendications

1. Procédé de préparation de dérivés d'acide α-cétocarboxylique, représentés par la formule 1 suivante, consistant à faire réagir des dérivés d'acide α-hydroxycarboxylique, représentés par la formule 2 suivante, avec un agent oxydant,
**caractérisée en ce que** l'agent oxydant est de l'acide hypobromeux, 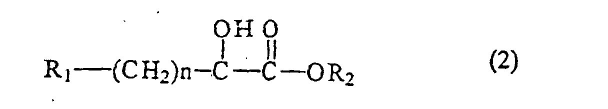 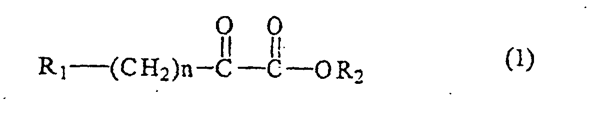 dans laquelle R₁ représente un atome d'hydrogène, un groupe alkyle en C₁-C₁₀, un groupe haloalkyle en C₁-C₁₀, un groupe alkoxyalkyle en C₁-C₁₀, un groupe cycloalkyle en C₃-C₆, ou un groupe aryle, dans lequel ledit groupe aryle est benzène, naphtalène, pyridine ou thiophène, le groupe aryle portant plus d'un substituant choisi parmi l'atome d'hydrogène et les groupes fonctionnels hydroxy, atome d'halogène, alkyle en C₁-C₃, -(CH₂)ₘL, alkoxy en C₁-C₃, phénoxy et nitro, dans lesquels ledit L est un atome d'halogène, un groupe alkylsulfonyloxy, benzènesulfonyloxy ou toluènesulfonyloxy, et ledit m est un nombre entier égal à 1 ou 2 ; R₂ représente un atome d'hydrogène ou un groupe alkyle en C₁-C₆ ; et n représente 0 ou un nombre entier de 1 à 10.

2. Procédé selon la revendication 1, dans lequel ledit acide hypobromeux est utilisé dans la plage de 1-2 équivalents molaires par rapport au produit de départ représenté par la formule 2.

3. Procédé selon la revendication 1 ou 2, dans lequel ledit acide hypobromeux est utilisé dans la plage de 1,05-1,3 équivalents molaires par rapport au produit de départ représenté par la formule 2.

4. Procédé selon une ou plusieurs des revendications précédentes, dans lequel la réaction est effectuée dans la plage de température de -5∼40°C.

5. Procédé selon une ou plusieurs des revendications précédentes, dans lequel le composé représenté par la formule 1 est l'ester d'éthyle de l'acide pyruvique.

6. Procédé selon une ou plusieurs des revendications 1 à 4, dans lequel le composé représenté par la formule 1 est l'ester de butyle de l'acide pyruvique.

7. Procédé selon une ou plusieurs des revendications 1 à 4, dans lequel le composé représenté par la formule 1 est l'ester de méthyle de l'acide (2-méthylphényle)-glyoxylique.

8. Procédé selon une ou plusieurs des revendications 1 à 4, dans lequel le composé représenté par la formule 1 est l'ester d'éthyle de l'acide (2-chlorométhylphényle)-glyoxylique.

9. Procédé selon une ou plusieurs des revendications 1 à 4, dans lequel le composé représenté par la formule 1 est l'ester d'éthyle de l'acide (2-hydroxyphényle)-glyoxylique.

10. Procédé selon une ou plusieurs des revendications 1 à 4, dans lequel le composé représenté par la formule 1 est l'ester d'éthyle de l'acide (7-chlorométhylnaphtyle)-glyoxylique.
